(19) Europäisches Patentamt
European Patent Office
Office européen des brevets

(11) Veröffentlichungsnummer: **0 050 283**
**B1**

(12) # EUROPÄISCHE PATENTSCHRIFT

(45) Veröffentlichungstag der Patentschrift: **22.02.84**

(51) Int. Cl.³: **C 07 C 161/00**, C 07 C 131/105, A 01 N 47/24

(21) Anmeldenummer: **81108184.3**

(22) Anmeldetag: **12.10.81**

(54) **Hydroxamsäureester, Verfahren zu ihrer Herstellung, ihre Verwendung in Schädlingsbekämpfungsmitteln und Zwischenprodukte dafür.**

(30) Priorität: **17.10.80 DE 3039269**

(43) Veröffentlichungstag der Anmeldung: **28.04.82 Patentblatt 82/17**

(45) Bekanntmachung des Hinweises auf die Patenterteilung: **22.02.84 Patentblatt 84/8**

(84) Benannte Vertragsstaaten: **AT BE CH DE FR GB IT LI NL SE**

(56) Entgegenhaltungen:
**DE - A - 2 111 459**
**FR - A - 2 351 099**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Schmetzer, Johannes, Dr., Jakob-Böhme-Strasse 21, D-5000 Köln 80 (DE)**
Erfinder: **Stetter, Jörg, Dr., Gellertweg 4, D-5600 Wuppertal 1 (DE)**
Erfinder: **Hammann, Ingeborg, Dr., Belfortstrasse 9, D-5000 Koeln (DE)**
Erfinder: **Homeyer, Bernhard, Dr., Obere Strasse 28, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Hydroxamsäureester, Verfahren zu ihrer Herstellung, ihre Verwendung in Schädlingsbekämpfungsmitteln und Zwischenprodukte dafür

Die vorliegende Erfindung betrifft neue Hydroxamsäureester, Verfahren zu ihrer Herstellung, ihre Verwendung in Schädlingsbekämpfungsmitteln, sowie Zwischenprodukte zu ihrer Herstellung und Verfahren zur Herstellung dieser Zwischenprodukte.

Es ist bereits bekannt geworden, daß Methylthio-substituierte Oximcarbamate wie z. B. 3,3-Dimethyl-2-methyl-carbamoyloximino-1-methylthio-butan (vgl. deutsche Offenlegungsschrift 2 216 838) insektizide und akarizide Eigenschaften besitzen. Es ist außerdem bekannt geworden, daß das Bis-Methylcarbamat von Methylthio-1-propandion-dioxim-1,2 insektizide Wirksamkeit aufweist (vgl. franz. Anm. 2 351 099). Diese befriedigen jedoch vor allem bei niedrigen Aufwandkonzentrationen nicht voll.

1. Es wurden die neuen Hydroxamsäureester der Formel I gefunden,

$$\begin{array}{l} R^1-C{=}N-OR^4 \\ \quad\;\;| \\ R^2X-C{=}N-OR^3 \end{array} \qquad (I)$$

in welcher

$R^1$ für gegebenenfalls substituiertes Alkyl, Cycloalkyl, Aryl, Aralkyl steht,
$R^2$ für Alkyl, Alkenyl, Alkinyl steht,
$R^1$ und $R^2$ gemeinsam für eine Alkylenbrücke mit 2—4 C-Atomen stehen und
einer der Reste $R^3$ oder $R^4$ für den Rest

$$R^5-NH-\overset{\overset{\displaystyle O}{\|}}{C}-$$

steht, während der andere Rest $R^3$ oder $R^4$ für Alkyl, Alkenyl, Alkinyl, Halogenalkyl, Halogenalkenyl oder Alkoxyalkyl steht,
$R^5$ für Alkyl steht und
X für O oder S steht.

2. Es wurde gefunden, daß die Hydroxamsäureester der Formel I hergestellt werden können, indem man Oxime der Formel II

$$\begin{array}{l} R^1-C{=}N-O-R^6 \\ \quad\;\;| \\ R^2X-C{=}N-O-R^7 \end{array} \qquad (II)$$

in welcher

$R^1$, $R^2$ und X die oben angegebenen Bedeutungen haben und
einer der Reste $R^6$ oder $R^7$ für H steht und der andere Rest $R^6$ oder $R^7$ für Alkyl, Alkenyl, Alkinyl, Halogenalkyl, Halogenalkenyl oder Alkoxyalkyl steht

mit Isocyanaten der Formel III

$$R^5NCO \qquad (III)$$

in welcher

$R^5$ die oben angegebene Bedeutung hat,

umsetzt.

3. Es wurden die neuen Oxime der Formel II gefunden,

$$\begin{array}{l} R^1-C{=}N-OR^6 \\ \quad\;\;| \\ R^2X-C{=}N-OR^7 \end{array} \qquad (II)$$

in welcher

$R^1$, $R^2$, X, $R^6$, $R^7$ die oben angegebenen Bedeutungen haben.

4. Es wurde gefunden, daß die Oxime der Formel II hergestellt werden können, indem man Ketone der Formel IV

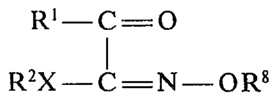

(IV)

in welcher

$R^1$, $R^2$ und X die oben angegebenen Bedeutungen haben und
$R^8$ für Wasserstoff oder für die unter $R^7$ angegebenen Reste steht,

a) für den Fall, daß $R^8$ für Wasserstoff steht, mit Hydroxylaminethern bzw. deren Salzen der Formel V

$R^6ONH_2XHY$ (V)

in welcher

$R^6$ die oben angegebene Bedeutung hat und
Y für das Anion einer Säure steht

umsetzt, oder
b) für den Fall, daß $R^8$ für die unter $R^7$ angegebenen Reste steht, mit Hydroxylamin bzw. seinen Salzen umsetzt.

Es wurden die Ketone der Formel IV gefunden

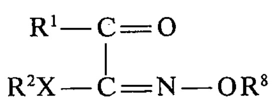

(IV)

in welcher

$R^1$, $R^2$, X und $R^8$ die oben angegebenen Bedeutungen haben.

Es wurde gefunden, daß die Ketone der Formel IV hergestellt werden können, indem man

a) für den Fall, daß $R^8$ für Wasserstoff steht, Ketone der Formel VI

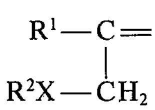

(VI)

in welcher

$R^1$, $R^2$ und X die oben angegebenen Bedeutungen haben,

mit Nitrosierungsmitteln umsetzt, und
b) für den Fall, daß $R^8$ für die unter $R^7$ angegebenen Reste steht, die bei Verfahren (6a) erhältlichen Verbindungen der Formel VII

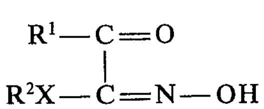

(VII)

in welcher

$R^1$, $R^2$ und X die oben angegebenen Bedeutungen haben,

mit Verbindungen der Formel VIII

$R^7W$ (VIII)

in welcher

$R^7$ die oben angegebene Bedeutung hat und
W für Cl, Br oder $R^7OSO_2O$ steht,

umsetzt.

Die Verbindungen der Formel (I) können in der syn- oder anti-Form vorliegen; vorwiegend fallen sie als Gemisch beider Formen an.

Die Verbindungen der Formel (I) eignen sich zur Schädlingsbekämpfung; insbesondere besitzen sie starke insektizide, akarizide und nematizide Eigenschaften.

Überraschenderweise zeigen die Verbindungen der Formel (I) eine höhere insektizide und akarizide Wirkung als das bekannte 3,3-Dimethyl-2-methylcarbamoyloximino-1-methylthio-butan.

Die erfindungsgemäßen Hydroxamsäureester sind durch die Formel (I) allgemein definiert. In dieser Formel steht $R^1$ vorzugsweise für Alkyl mit 1 bis 4 Kohlenstoffatomen, Halogenalkyl mit 1 bis 4 Kohlenstoffatomen und bis zu 5 Halogenatomen wie insbesondere Fluor, Chlor oder Brom, Cycloalkyl mit bis zu 8 Kohlenstoffatomen, Alkoxyalkyl und Alkylthioalkyl mit bis zu 3 C-Atomen in jedem Alkylteil, gegebenenfalls substituiertes Phenyl, wobei als Substituenten vorzugsweise genannt seien: Halogen, insbesondere Fluor, Chlor, Brom, Cyano, Nitro, Alkyl mit 1 bis 2 Kohlenstoffatomen. $R^2$ steht vorzugsweise für Alkyl mit 1 bis 4 Kohlenstoffatomen, Alkenyl mit 1 bis 4 Kohlenstoffatomen und Alkinyl mit 1 bis 4 Kohlenstoffatomen. $R^1$ und $R^2$ stehen auch gemeinsam für eine gegebenenfalls methylsubstituierte Methylen-, Ethylen- oder Propylen-Brücke. $R^3$ oder $R^4$ stehen vorzugsweise für Alkyl mit 1 bis 4 Kohlenstoffatomen, Alkenyl mit 2 bis 4 Kohlenstoffatomen, Alkinyl mit 2 bis 4 Kohlenstoffatomen, Halogenalkyl mit 1 bis 4 Kohlenstoffatomen und bis zu 3 Halogenatomen, Halogenalkenyl mit 2 bis 4 Kohlenstoffatomen und bis zu 3 Halogenatomen, Alkoxyalkyl mit bis zu 2 Kohlenstoffatomen in jedem Alkylteil und den Rest

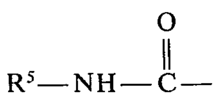

(es darf dabei nur jeweils einer der Reste $R^3$ oder $R^4$ für den Rest

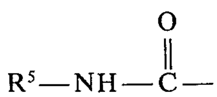

stehen). $R^5$ steht vorzugsweise für Methyl oder Ethyl. X steht vorzugsweise für S.

Ganz besonders bevorzugt sind diejenigen Hydroxamsäureester der Formel (I), in denen $R^1$ für Methyl, Ethyl, Propyl, Isopropyl, tert.-Butyl, Fluor-, Chlor-, Brom-, Difluor-, Dichlor-tert.-Butyl steht; $R^2$ für Methyl oder Ethyl steht; $R^1$ und $R^2$ für eine Ethylenbrücke stehen; $R^3$ oder $R^4$ für Methyl, Ethyl, Propargyl, Allyl oder Methylcarbamoyl stehen (wobei nur einer der Reste $R^3$ oder $R^4$ für Methylcarbamoyl stehen darf); und X für S steht.

Im einzelnen seien außer den Herstellungsbeispielen und den Beispielen der Tabelle die folgenden Verbindungen genannt.

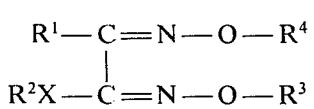

(I)

| $R^1$ | $R^2$ | $R^3$ | $R^4$ | X |
|---|---|---|---|---|
| $(CH_3)_3C—$ | $CH_3$ | $CH_3$ | $CH_3NH—CO—$ | S |
| desgl. | $CH_3$ | $C_2H_5$ | desgl. | S |
| desgl. | $CH_3$ | $C_3H_7$ | desgl. | S |
| desgl. | $CH_3$ | $—CH_2—C{\equiv}CH$ | desgl. | S |
| desgl. | $CH_3$ | $—CH_2—CH{=}CH_2$ | desgl. | S |
| desgl. | $CH_3$ | $—CH_2—C(CH_3){=}CH_2$ | desgl. | S |

(Fortsetzung)

| $R^1$ | $R^2$ | $R^3$ | $R^4$ | X |
|---|---|---|---|---|
| $(CH_3)_3C-$ | $C_2H_5$ | $CH_3$ | $CH_3NH-CO-$ | S |
| desgl. | $C_2H_5$ | $C_2H_5$ | desgl. | S |
| desgl. | $C_2H_5$ | $C_3H_7$ | desgl. | S |
| desgl. | $C_2H_5$ | $-CH_2-C\equiv CH$ | desgl. | S |
| desgl. | $C_2H_5$ | $-CH_2-CH=CH_2$ | desgl. | S |
| desgl. | $C_3H_7$ | $CH_3$ | desgl. | S |
| desgl. | $C_3H_7$ | $C_2H_5$ | desgl. | S |
| desgl. | $-CH_2-CH=CH_2$ | $CH_3$ | desgl. | S |
| desgl. | desgl. | $C_2H_5$ | desgl. | S |
| $R^1 + R^2$ | | | | |
| 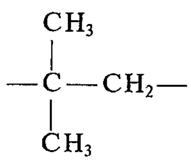 | | $CH_3$ | desgl. | S |
| desgl. | | $C_2H_5$ | desgl. | S |
| desgl. | | $-CH_2-C\equiv CH$ | desgl. | S |
| desgl. | | $-CH_2-CH=CH_2$ | desgl. | S |
| $R^1 + R^2$ | | | | |
| $-CH_2-CH_2-$ | | $CH_3$ | desgl. | S |
| desgl. | | $C_2H_5$ | desgl. | S |
| desgl. | | $-CH-C\equiv CH$ | desgl. | S |
| desgl. | | $-CH_2-CH=CH_2$ | desgl. | S |
| $R^1 + R^2$ | | | | |
| $-CH_2-CH_2-CH_2-$ | | $CH_3$ | desgl. | S |
| desgl. | | $C_2H_5$ | desgl. | S |
| desgl. | | $-CH_2-C\equiv CH$ | desgl. | S |
| desgl. | | $-CH_2-CH=CH_2$ | desgl. | S |
| $(CH_3)_2HC-$ | $CH_3$ | $CH_3$ | desgl. | S |
| desgl. | $CH_3$ | $C_2H_5$ | desgl. | S |
| desgl. | $CH_3$ | $C_3H_7$ | desgl. | S |
| desgl. | $CH_3$ | $-CH_2-C\equiv CH$ | desgl. | S |
| desgl. | $CH_3$ | $-CH_2-CH=CH_2$ | desgl. | S |
| desgl. | $CH_3$ | $-CH_2-C(CH_3)=CH_2$ | desgl. | S |
| desgl. | $C_2H_5$ | $CH_3$ | desgl. | S |
| desgl. | $C_2H_5$ | $C_2H_5$ | desgl. | S |
| desgl. | $C_2H_5$ | $C_3H_7$ | desgl. | S |

(Fortsetzung)

| $R^1$ | $R^2$ | $R^3$ | $R^4$ | X |
|---|---|---|---|---|
| $(CH_3)_2HC-$ | $C_2H_5$ | $-CH_2-C{\equiv}CH$ | $CH_3NH-CO-$ | S |
| desgl. | $C_2H_5$ | $-CH_2-CH{=}CH_2$ | desgl. | S |
| desgl. | $C_3H_7$ | $CH_3$ | desgl. | S |
| desgl. | $C_3H_7$ | $C_2H_5$ | desgl. | S |
| desgl. | $-CH_2-CH{=}CH_2$ | $CH_3$ | desgl. | S |
| desgl. | desgl. | $C_2H_5$ | desgl. | S |
| 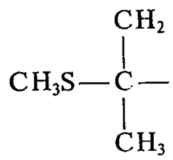 | $CH_3$ | $CH_3$ | desgl. | S |
| desgl. | $CH_3$ | $C_2H_5$ | desgl. | S |
| desgl. | $CH_3$ | $C_3H_7$ | desgl. | S |
| desgl. | $CH_3$ | $-CH_2-C{\equiv}CH$ | desgl. | S |
| desgl. | $CH_3$ | $-CH_2-CH{=}CH_2$ | desgl. | S |
| desgl. | $CH_3$ | $-CH_2-C(CH_3){=}CH_2$ | desgl. | S |
| desgl. | $C_2H_5$ | $CH_3$ | desgl. | S |
| desgl. | $C_2H_5$ | $C_2H_5$ | desgl. | S |
| desgl. | $C_2H_5$ | $C_3H_7$ | desgl. | S |
| desgl. | $C_2H_5$ | $-CH_2-C{\equiv}CH$ | desgl. | S |
| desgl. | $C_2H_5$ | $-CH_2-CH{=}CH_2$ | desgl. | S |
| desgl. | $C_3H_7$ | $CH_3$ | desgl. | S |
| desgl. | $C_3H_7$ | $C_2H_5$ | desgl. | S |
| desgl. | $-CH_2-CH{=}CH_2$ | $CH_3$ | desgl. | S |
| desgl. | desgl. | $C_2H_5$ | desgl. | S |
| 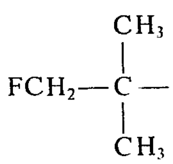 | $CH_3$ | $CH_3$ | desgl. | S |
| desgl. | $CH_3$ | $C_2H_5$ | desgl. | S |
| desgl. | $CH_3$ | $C_3H_7$ | desgl. | S |
| desgl. | $CH_3$ | $-CH_2-C{\equiv}CH$ | desgl. | S |
| desgl. | $CH_3$ | $-CH_2-CH{=}CH_2$ | desgl. | S |
| desgl. | $CH_3$ | $-CH_2-C(CH_3){=}CH_2$ | desgl. | S |
| desgl. | $C_2H_5$ | $CH_3$ | desgl. | S |
| desgl. | $C_2H_5$ | $C_2H_5$ | desgl. | S |
| desgl. | $C_2H_5$ | $C_3H_7$ | desgl. | S |
| desgl. | $C_2H_5$ | $-CH_2-C{\equiv}CH$ | desgl. | S |
| desgl. | $C_2H_5$ | $-CH_2-CH{=}CH_2$ | desgl. | S |

(Fortsetzung)

| $R^1$ | $R^2$ | $R^3$ | $R^4$ | X |
|---|---|---|---|---|
| 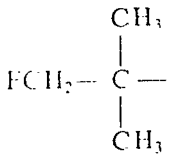 | $C_3H_7$ | $CH_3$ | $CH_3NH-CO-$ | S |
| desgl. | $C_3H_7$ | $C_2H_5$ | desgl. | S |
| desgl. | $-CH_2-CH{=}CH_2$ | $CH_3$ | desgl. | S |
| desgl. | desgl. | $C_2H_5$ | desgl. | S |
| 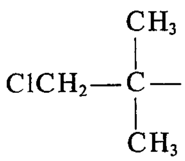 | $CH_3$ | $CH_3$ | desgl. | S |
| desgl. | $CH_3$ | $C_2H_5$ | desgl. | S |
| desgl. | $CH_3$ | $C_3H_7$ | desgl. | S |
| desgl. | $CH_3$ | $-CH_2-C{\equiv}CH$ | desgl. | S |
| desgl. | $CH_3$ | $-CH_2-CH{=}CH_2$ | desgl. | S |
| desgl. | $CH_3$ | $-CH_2-C(CH_3){=}CH_2$ | desgl. | S |
| desgl. | $C_2H_5$ | $CH_3$ | desgl. | S |
| desgl. | $C_2H_5$ | $C_2H_5$ | desgl. | S |
| desgl. | $C_2H_5$ | $C_3H_7$ | desgl. | S |
| desgl. | $C_2H_5$ | $-CH_2-C{\equiv}CH$ | desgl. | S |
| desgl. | $C_2H_5$ | $-CH_2-CH{=}CH_2$ | desgl. | S |
| desgl. | $C_3H_7$ | $CH_3$ | desgl. | S |
| desgl. | $C_3H_7$ | $C_2H_5$ | desgl. | S |
| desgl. | $-CH_2-CH{=}CH_2$ | $CH_3$ | desgl. | S |
| desgl. | desgl. | $C_2H_5$ | desgl. | S |
| 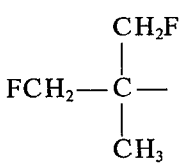 | $CH_3$ | $CH_3$ | desgl. | S |
| desgl. | $CH_3$ | $C_2H_5$ | desgl. | S |
| desgl. | $CH_3$ | $C_3H_7$ | desgl. | S |
| desgl. | $CH_3$ | $-CH_2-C{\equiv}CH$ | desgl. | S |
| desgl. | $CH_3$ | $-CH_2-CH{=}CH_2$ | desgl. | S |
| desgl. | $CH_3$ | $-CH_2-C(CH_3){=}CH_2$ | desgl. | S |
| desgl. | $C_2H_5$ | $CH_3$ | desgl. | S |
| desgl. | $C_2H_5$ | $C_2H_5$ | desgl. | S |
| desgl. | $C_2H_5$ | $C_3H_7$ | desgl. | S |
| desgl. | $C_2H_5$ | $-CH_2-C{\equiv}CH$ | desgl. | S |

(Fortsetzung)

| $R^1$ | $R^2$ | $R^3$ | $R^4$ | X |
|---|---|---|---|---|
| 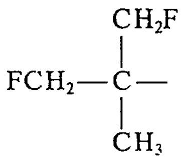 | $C_2H_5$ | $-CH_2-CH=CH_2$ | $CH_3NH-CO-$ | S |
| desgl. | $C_3H_7$ | $CH_3$ | desgl. | S |
| desgl. | $C_3H_7$ | $C_2H_5$ | desgl. | S |
| desgl. | $-CH_2-CH=CH_2$ | $CH_3$ | desgl. | S |
| desgl. | desgl. | $C_2H_5$ | desgl. | S |
| $CH_3$ | $CH_3$ | $CH_3$ | desgl. | S |
| $CH_3$ | $CH_3$ | $C_2H_5$ | desgl. | S |
| $CH_3$ | $CH_3$ | $C_3H_7$ | desgl. | S |
| $CH_3$ | $CH_3$ | $-CH_2-C\equiv CH$ | desgl. | S |
| $CH_3$ | $CH_3$ | $-CH_2-CH=CH_2$ | desgl. | S |
| $CH_3$ | $CH_3$ | $-CH_2-C(CH_3)=CH_2$ | desgl. | S |
| $CH_3$ | $C_2H_5$ | $CH_3$ | desgl. | S |
| $CH_3$ | $C_2H_5$ | $C_2H_5$ | desgl. | S |
| $CH_3$ | $C_2H_5$ | $C_3H_7$ | desgl. | S |
| $CH_3$ | $C_2H_5$ | $-CH_2-C\equiv CH$ | desgl. | S |
| $CH_3$ | $C_2H_5$ | $-CH_2-CH=CH_2$ | desgl. | S |
| $CH_3$ | $C_3H_7$ | $CH_3$ | desgl. | S |
| $CH_3$ | $C_3H_7$ | $C_2H_5$ | desgl. | S |
| $CH_3$ | $-CH_2-CH=CH_2$ | $CH_3$ | desgl. | S |
| $CH_3$ | desgl. | $C_2H_5$ | desgl. | S |
| $(CH_3)_3C-$ | $CH_3$ | $CH_3NH-CO-$ | $CH_3$ | S |
| desgl. | $CH_3$ | desgl. | $C_2H_5$ | S |
| desgl. | $CH_3$ | desgl. | $-CH_2-C\equiv CH$ | S |
| desgl. | $CH_3$ | desgl. | $-CH_2-CH=CH_2$ | S |
| desgl. | $C_2H_5$ | desgl. | $CH_3$ | S |
| desgl. | $C_2H_5$ | desgl. | $C_2H_5$ | S |
| desgl. | $C_2H_5$ | desgl. | $-CH_2-C\equiv CH$ | S |
| desgl. | $C_2H_5$ | desgl. | $-CH_2-CH=CH_2$ | S |
| desgl. | $C_3H_7$ | desgl. | $CH_3$ | S |
| desgl. | $C_3H_7$ | desgl. | $C_2H_5$ | S |
| desgl. | $C_3H_7$ | desgl. | $-CH_2-C\equiv CH$ | S |
| desgl. | $C_3H_7$ | desgl. | $-CH_2-CH=CH_2$ | S |
| desgl. | $-CH_2-CH=CH_2$ | desgl. | $CH_3$ | S |
| desgl. | desgl. | desgl. | $C_2H_5$ | S |

(Fortsetzung)

| $R^1$ | $R^2$ | $R^3$ | $R^4$ | X |
|---|---|---|---|---|
| $R^1 + R^2$ | | | | |
| 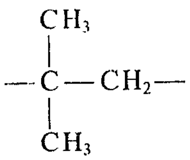 | | $CH_3NH—CO—$ | $CH_3$ | S |
| desgl. | | desgl. | $C_2H_5$ | S |
| desgl. | | desgl. | $—CH_2—C{\equiv}CH$ | S |
| desgl. | | desgl. | $—CH_2—CH{=}CH_2$ | S |
| $R^1 + R^2$ | | | | |
| $—CH_2—CH_2—$ | | desgl. | $CH_3$ | S |
| desgl. | | desgl. | $C_2H_5$ | S |
| desgl. | | desgl. | $—CH_2—C{\equiv}CH$ | S |
| desgl. | | desgl. | $—CH_2—CH{=}CH_2$ | S |
| $R^1 + R^2$ | | | | |
| $—CH_2—CH_2—CH_2—$ | | desgl. | $CH_3$ | S |
| desgl. | | desgl. | $C_2H_5$ | S |
| desgl. | | desgl. | $—CH_2—C{\equiv}CH$ | S |
| desgl. | | desgl. | $—CH_2—CH{=}CH_2$ | S |
| $(CH_3)_2HC—$ | $CH_3$ | desgl. | $CH_3$ | S |
| desgl. | $CH_3$ | desgl. | $C_2H_5$ | S |
| desgl. | $CH_3$ | desgl. | $—CH_2—C{\equiv}CH$ | S |
| desgl. | $CH_3$ | desgl. | $—CH_2—CH{=}CH_2$ | S |
| desgl. | $C_2H_5$ | desgl. | $CH_3$ | S |
| desgl. | $C_2H_5$ | desgl. | $C_2H_5$ | S |
| desgl. | $C_2H_5$ | desgl. | $—CH_2—C{\equiv}CH$ | S |
| desgl. | $C_2H_5$ | desgl. | $—CH_2—CH{=}CH_2$ | S |
| desgl. | $C_3H_7$ | desgl. | $CH_3$ | S |
| desgl. | $C_3H_7$ | desgl. | $C_2H_5$ | S |
| desgl. | $C_3H_7$ | desgl. | $—CH_2—C{\equiv}CH$ | S |
| desgl. | $C_3H_7$ | desgl. | $—CH_2—CH{=}CH_2$ | S |
| desgl. | $—CH_2—CH{=}CH_2$ | desgl. | $CH_3$ | S |
| desgl. | desgl. | desgl. | $C_2H_5$ | S |
| 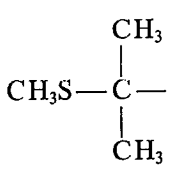 | $CH_3$ | desgl. | $CH_3$ | S |
| desgl. | $CH_3$ | desgl. | $C_2H_5$ | S |

(Fortsetzung)

| $R^1$ | $R^2$ | $R^3$ | $R^4$ | X |
|---|---|---|---|---|
| 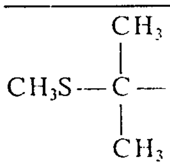 | $CH_3$ | $CH_3NH-CO-$ | $-CH_2-C\equiv CH$ | S |
| desgl. | $CH_3$ | desgl. | $-CH_2-CH=CH_2$ | S |
| desgl. | $C_2H_5$ | desgl. | $CH_3$ | S |
| desgl. | $C_2H_5$ | desgl. | $C_2H_5$ | S |
| desgl. | $C_2H_5$ | desgl. | $-CH_2-C\equiv CH$ | S |
| desgl. | $C_2H_5$ | desgl. | $-CH_2-CH=CH_2$ | S |
| desgl. | $C_3H_7$ | desgl. | $CH_3$ | S |
| desgl. | $C_3H_7$ | desgl. | $C_2H_5$ | S |
| desgl. | $-CH_2-CH=CH_2$ | desgl. | $CH_3$ | S |
| desgl. | desgl. | desgl. | $C_2H_5$ | S |
| 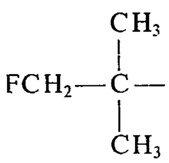 | $CH_3$ | desgl. | $CH_3$ | S |
| desgl. | $CH_3$ | desgl. | $C_2H_5$ | S |
| desgl. | $CH_3$ | desgl. | $-CH_2-C\equiv CH$ | S |
| desgl. | $CH_3$ | desgl. | $-CH_2-CH=CH_2$ | S |
| desgl. | $C_2H_5$ | desgl. | $CH_3$ | S |
| desgl. | $C_2H_5$ | desgl. | $C_2H_5$ | S |
| desgl. | $C_2H_5$ | desgl. | $-CH_2-C\equiv CH$ | S |
| desgl. | $C_2H_5$ | desgl. | $-CH_2-CH=CH_2$ | S |
| desgl. | $C_3H_7$ | desgl. | $CH_3$ | S |
| desgl. | $C_3H_7$ | desgl. | $C_2H_5$ | S |
| desgl. | $-CH_2-CH=CH_2$ | desgl. | $CH_3$ | S |
| desgl. | desgl. | desgl. | $C_2H_5$ | S |
| 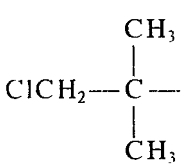 | $CH_3$ | desgl. | $CH_3$ | S |
| desgl. | $CH_3$ | desgl. | $C_2H_5$ | S |
| desgl. | $CH_3$ | desgl. | $-CH_2-C\equiv CH$ | S |
| desgl. | $CH_3$ | desgl. | $-CH_2-CH=CH_2$ | S |
| desgl. | $C_2H_5$ | desgl. | $CH_3$ | S |
| desgl. | $C_2H_5$ | desgl. | $C_2H_5$ | S |
| desgl. | $C_2H_5$ | desgl. | $-CH_2-C\equiv CH$ | S |

(Fortsetzung)

| $R^1$ | $R^2$ | $R^3$ | $R^4$ | X |
|---|---|---|---|---|
| 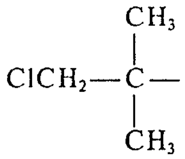 | $C_2H_5$ | $CH_3NH-CO-$ | $-CH_2-CH=CH_2$ | S |
| desgl. | $C_3H_7$ | desgl. | $CH_3$ | S |
| desgl. | $C_3H_7$ | desgl. | $C_2H_5$ | S |
| desgl. | $-CH_2-CH=CH_2$ | desgl. | $CH_3$ | S |
| desgl. | desgl. | desgl. | $C_2H_5$ | S |
| 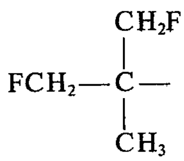 | $CH_3$ | desgl. | $CH_3$ | S |
| desgl. | $CH_3$ | desgl. | $C_2H_5$ | S |
| desgl. | $CH_3$ | desgl. | $-CH_2-C\equiv CH$ | S |
| desgl. | $CH_3$ | desgl. | $-CH_2-CH=CH_2$ | S |
| desgl. | $C_2H_5$ | desgl. | $CH_3$ | S |
| desgl. | $C_2H_5$ | desgl. | $C_2H_5$ | S |
| desgl. | $C_2H_5$ | desgl. | $-CH_2-C\equiv CH$ | S |
| desgl. | $C_2H_5$ | desgl. | $-CH_2-CH=CH_2$ | S |
| desgl. | $C_3H_7$ | desgl. | $CH_3$ | S |
| desgl. | $C_3H_7$ | desgl. | $C_2H_5$ | S |
| desgl. | $-CH_2-CH=CH_2$ | desgl. | $CH_3$ | S |
| desgl. | desgl. | desgl. | $C_2H_5$ | S |
| $CH_3$ | $CH_3$ | desgl. | $CH_3$ | S |
| $CH_3$ | $CH_3$ | desgl. | $C_2H_5$ | S |
| $CH_3$ | $CH_3$ | desgl. | $-CH_2-C=CH$ | S |
| $CH_3$ | $CH_3$ | desgl. | $-CH_2-CH=CH_2$ | S |
| $CH_3$ | $C_2H_5$ | desgl. | $CH_3$ | S |
| $CH_3$ | $C_2H_5$ | desgl. | $C_2H_5$ | S |
| $CH_3$ | $C_2H_5$ | desgl. | $-CH_2-C=CH$ | S |
| $CH_3$ | $C_2H_5$ | desgl. | $-CH_2-CH=CH_2$ | S |
| $CH_3$ | $C_3H_7$ | desgl. | $CH_3$ | S |
| $CH_3$ | $C_3H_7$ | desgl. | $C_2H_5$ | S |
| $CH_3$ | $C_3H_7$ | desgl. | $-CH_2-C=CH$ | S |
| $CH_3$ | $C_3H_7$ | desgl. | $-CH_2-CH=CH_2$ | S |
| $CH_3$ | $-CH_2-CH=CH_2$ | desgl. | $CH_3$ | S |
| $CH_3$ | desgl. | desgl. | $C_2H_5$ | S |

Die Hydroxamsäureester der Formel I werden durch Umsetzung der Oxime der Formel II mit Isocyanaten der Formel III hergestellt.

Verwendet man 1-Methoximino-1-methylmercapto-2-hydroxyimino-3,3-dimethyl-butan und Methylisocyanat als Ausgangsstoffe, so kann der Reaktionsablauf durch das folgende Formelschema wiedergegeben werden.

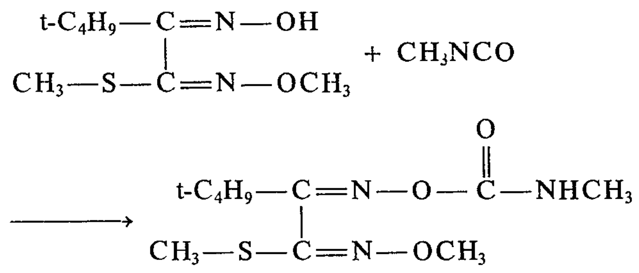

Verwendet man 2-Methoximino-1-methylmercapto-1-hydroximinopropan und Methylisocyanat als Ausgangsstoffe, so kann der Reaktionsablauf durch das folgende Formelschema wiedergegeben werden:

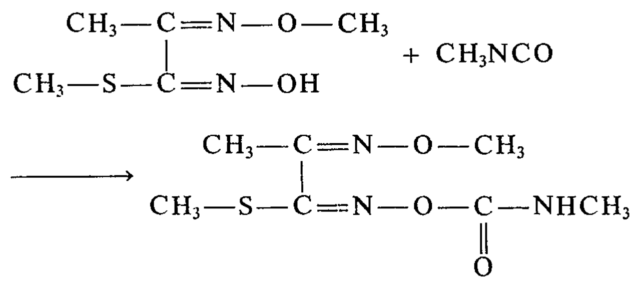

Die Herstellung der Verbindung der Formel I erfolgt indem man die Oxime der Formel II mit Isocyanaten der Formel III gegebenenfalls in einem inerten organischen Verdünnungsmittel und unter Zusatz eines Katalysators wie Triethylamin oder einer organischen Zinnverbindung wie Dibutyl-Zinn-dilaureat umsetzt.

Als Verdünnungsmittel seien genannt Kohlenwasserstoffe wie Benzol, Toluol, Cyclohexan oder Petrolether, halogenierte Kohlenwasserstoffe wie z.B. Chloroform, Methylenchlorid oder Chlorbenzol, Ether wie Diethylether, Dioxan oder Tetrahydrofuran und Ketone wie Aceton oder Methylisobutylketon sowie Essigester.

Die Herstellung erfolgt in einem molaren Verhältnis der Oxime der Formel II zu den Isocyanaten der Formel III von 1 : 1. Bevorzugt kann auch ein Überschuß der Isocyanate der Formel III verwendet werden.

Die Umsetzung erfolgt bei Temperaturen von 0 bis 100° C und einem Druck von 1 bar.

Die Oxime der Formel II sind neu. Bevorzugte Oxime der Formel II sind solche deren Reste $R^1$, $R^2$ und X die bei den Verbindungen der Formel I angegebene bevorzugte und besonders bevorzugte Bedeutung besitzen.

$R^6$ oder $R^7$ stehen bevorzugt für $C_{1-4}$-Alkyl, $C_{2-4}$-Alkenyl, $C_{2-4}$-Alkinyl, $C_{1-4}$-Halogenalkyl, mit bis zu 3 Halogenatomen, Alkoxyalkyl mit bis zu 2 C-Atomen in jedem Alkylteil, insbesondere stehen sie für Methyl, Ethyl,Propargyl oder Allyl.

Oxime der Formel II werden zum Teil erhalten, indem man Ketone der Formel IV für den Fall, daß $R^8$ für Wasserstoff steht, mit Hydroxylaminethern bzw. deren Salze der Formel V in Gegenwart eines organischen Lösungsmittels wie beispielsweise Ethanol und gegebenenfalls in Gegenwart einer Hilfsbase wie beispielsweise Natriumacetat bei Temperaturen zwischen 20° C und 120° C, vorzugsweise zwischen 50 und 100° C umsetzt. Die Isolierung der Verbindungen (II) erfolgt in der üblichen Weise, z. B. indem man das Reaktionsgemisch mit Wasser versetzt und den Niederschlag abfiltriert. Gegebenenfalls können die Oxime auch durch Umkristallisieren gereinigt werden.

Oxime der Formel II werden zum Teil erhalten indem man für den Fall, daß $R^8$ die unter $R^7$ angegebene Bedeutung hat, die Ketone der Formel IV mit Hydroxylamin bzw. seinen Salzen in Gegenwart eines organischen Lösungsmittels wie beispielsweise Ethanol und gegebenenfalls in Gegenwart einer Hilfsbase wie beispielsweise Natriumacetat bei Temperaturen zwischen 20° C und 120° C, vorzugsweise zwischen 50 und 100° C umsetzt. Die Isolierung der Verbindungen (II) erfolgt in der üblichen Weise, z. B. indem man das Reaktionsgemisch mit Wasser versetzt und den Niederschlag abfiltriert. Gegebenenfalls können die Oxime auch durch Umkristallisieren gereinigt werden.

Die Ketone der Formel IV sind teilweise ($R^8$ = H, vgl. DE-OS 2 111 459) neu. Bevorzugte Ketone der Formel IV sind solche der Reste $R^1$, $R^2$, $R^8$ und X die bei den Oximen der Formel II bzw. bei den

Hydroxamsäureestern der Formel I angegebenen bevorzugten und besonders bevorzugten Bedeutungen besitzen. Sie werden erhalten indem man für den Fall, daß $R^8$ für Wasserstoff steht, Ketone der Formel VI mit Nitrosierungsmitteln in Gegenwart einer Base wie beispielsweise Natriummethylat und in Gegenwart eines Verdünnungsmittels wie beispielsweise Ethanol oder Methanol versetzt.

Nach Ansäuren können die Ketoderivate der Formel IV in üblicher Weise isoliert werden.

Als Beispiele für geeignete Nitrosierungsmittel seien genannt: Nitrosylchlorid, Methylnitrit, Ethylnitrit, Propylnitrit, Isoamylnitrit.

Ketone und Nitrosierungsmittel werden in einem molaren Verhältnis von 1 : 1 eingesetzt.

Die Reaktion wird bei Temperaturen von 0 bis 100° C und einem Druck von 1 bis 2 bar durchgeführt.

Neue Ketone der Formel IV in welcher $R^8$ für die bei $R^7$ angegebenen Reste steht werden erhalten, indem man $\alpha$-Keto-Oxime der Formel VII mit Verbindungen der Formel VIII umsetzt.

Die $\alpha$-Keto-Oxime der Formel (VII) werden hierbei bevorzugt in Form ihrer Alkalisalze eingesetzt. Diese Alkalisalze können beispielsweise mit Hilfe einer Base wie Natriumhydrid in einem inerten Lösungsmittel wie Dimethylformamid oder mit Hilfe eines Alkalicarbonats wie Kaliumcarbonat in einem Lösungsmittel wie Aceton erzeugt werden. Eine bevorzugte Ausführungsform besteht darin, daß man die Ketone der Formel VII in einem Zweiphasensystem, wie beispielsweise wäßrige Natron- oder Kalilauge/Toluol oder Methylenchlorid, unter Zusatz von 0,1—1 Mol eines Phasen-Transfer-Katalysators, wie beispielsweise einer Ammonium- oder Phosponiumverbindung, beispielsweise sei Triethylbenzylammoniumchlorid genannt, mit Verbindungen der Formel VIII umsetzt.

Im allgemeinen arbeitet man zwischen 20 und 100° C, vorzugsweise zwischen 20 und 80° C.

Verbindungen der Formel VIII sind bekannte Alkylierungsmittel. Bevorzugt seien genannt: Dimethylsulfat, Diethylsulfat, Allylchlorid, Allylbromid, Propargylchlorid, Propargylbromid, Methallylchlorid.

Die Ketone der Formel VI sind zum Teil bekannt (vgl. DE-OS 2 216 838 und DE-PS 2 033 454).

Sie werden analog zu bekannten Methoden erhalten, z. B. indem man Verbindungen der Formel IX

$$R^1-\overset{\overset{\displaystyle O}{\|}}{C}-CH_2-W \qquad (IX)$$

in welcher

$R^1$ und W die oben angegebene Bedeutung besitzen

mit Verbindungen der Formel X

$$R^2XH \qquad (X)$$

in welcher

$R^2$ die oben angegebene Bedeutung besitzt, und
X für O oder S steht

umsetzt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp.
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata,

Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zelandica.
Aus der Ordnung der Hymenoptera z. B. Diprion spp., Hoplocampa spp., Lasius spp., Monomorium pharaonis, Vespa spp.
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp.
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.
Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberfächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

## Herstellungsbeispiele

### Beispiel 1

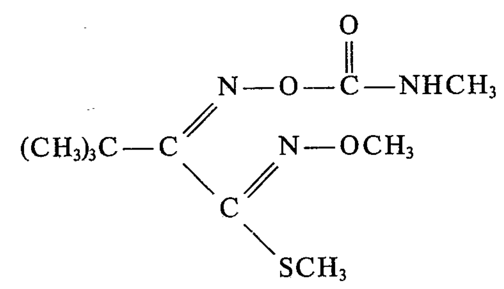

68,8 g (0,33 Mol) 3,3-Dimethyl-2-hydroximino-1-methoximino-1-methylmercapto-butan wurden in 250 ml Methylenchlorid unter Rühren tropfenweise mit 37,6 g (0,66 Mol) Methylisocyanat versetzt. Anschließend wurde noch eine Stunde am Rückfluß gekocht und das Lösungsmittel und überschüssiges Methylisocyanat im Vakuum entfernt. Der kristalline Rückstand wurde mit Petrolether verrührt und abgesaugt.

Ausbeute: 85,1 g (99% der Theorie)

Fp. 155–156° C.

Herstellung der Vorprodukte

a) 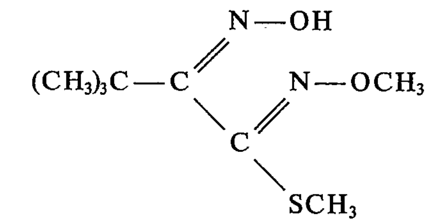

73,8 g (0,39 Mol) 3,3-Dimethyl-1-methoximino-1-methylmercapto-butan-2-on, 81,3 g (1,17 Mol) Hydroxylamin-Hydrochlorid und 95,9 g (1,17 Mol) Natriumacetat wurden in 500 ml Ethanol gelöst und 4 Stunden am Rückfluß gekocht. Nach Zugabe der gleichen Menge Hydroxylamin-Hydrochlorid und Natriumacetat wurde noch weitere 4 Stunden am Rückfluß gekocht und die Reaktionsmischung anschließend im Vakuum weitgehend vom Lösungsmittel befreit. Der Rückstand wurde mit Wasser versetzt, die entstandene Essigsäure mit Natriumhydrogencarbonat-Lösung neutralisiert und das Produkt mit Methylenchlorid extrahiert. Nach Trocknen der organischen Phase über Natriumsulfat wurde das Lösungsmittel im Vakuum abgezogen und der Rückstand nach Verreiben mit Diisopropylether abfiltriert.
Ausbeute: 57,1 g (72% der Theorie)
Fp. 137–138° C.

b) 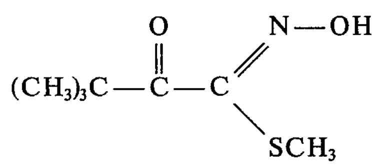

146 g (1 Mol) 1-Methylmercaptopinalkolim wurden mit 180 g 30%iger Natriummethylat-Lösung in Methanol vermischt und bei 0° C unter Kühlung mit 117,2 g Iso-Amylnitrit tropfenweise versetzt. Anschließend ließ man den Ansatz langsam auf Raumtemperatur kommen.

Es wurde noch 4 Stunden bei Raumtemperatur gerührt, die Hauptmenge Methanol im Vakuum entfernt, der Rückstand mit Wasser vernetzt, die wäßrige Lösung 3mal mit Chloroform ausgeschüttelt, die wäßrige Phase mit konzentrierter Salzsäure neutralisiert und das Produkt mit Methylenchlorid ausgeschüttelt. Nach Trocknen der organischen Phase über Natriumsulfat wurde das Lösungsmittel im Vakuum entfernt und der Rückstand mit Petrolether kristallisiert.
Ausbeute: 116 g (66% der Theorie)
Fp. 59–62° C.

Beispiel 2

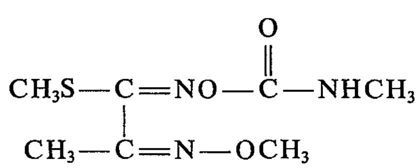

Zu einer Mischung aus 2,0 g (0,0123 Mol) 1-Methylthio-1-oximino-2-methoximino-propan und einem Überschuß von 2 g Methylisocyanat in 30 ml abs. Aceton wurden einige Tropfen Triethylamin gegeben. Nach 5 h Rühren bei Raumtemperatur wird das Lösungsmittel abdestilliert. Der Rückstand wurde in Chloroform aufgenommen und die organische Phase zweimal mit $H_2O$ ausgeschüttelt, über $Na_2SO_4$ getrocknet und eingeengt.
Ausbeute: 2,3 g (86% der Theorie)
$n_D^{20}$: 1,5330.

a) 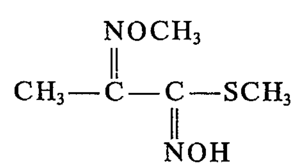

Eine Mischung aus 45 g (0,33 Mol) 1-Methylthio-1-oximino-propan-2-on, 56 g (0,67 Mol) Hydroxylaminmethylether-Hydrochlorid, 55 g (0,67 Mol) Natriumacetat und 400 ml Ethanol wurde 2 h am Rückfluß erhitzt.

Die Reaktionsmischung wurde auf die Hälfte eingeengt und mit verdünnter KOH aufgenommen. Man extrahiert zweimal mit $CHCl_3$ und stellte die wäßrige Lösung mit HCl auf pH 5–6 ein. Die $H_2O$-Phase wird dann dreimal mit $CHCl_3$ extrahiert, über $Na_2SO_4$ getrocknet und eingeengt. Destillation am Kugelrohrofen bei 150–170° C/0,3 Torr ergab ca. 40 g eines wasserklaren, zähen Öls, welches allmählich auskristallisiert.
Ausbeute: 70% der Theorie 1-Methylthio-1-oximino-2-methoximino-propan.
Fp: 52–74° C.

Analog Beispiel 1 und 2 wurden hergestellt:

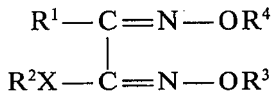

| Beispiel Nr. | $R^1$ | $R^2$ | $R^3$ | $R^4$ | X | Fp.: |
|---|---|---|---|---|---|---|
| 3 | $(CH_3)_3C$ | $CH_3$ | $C_2H_5$ | 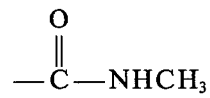 | S | 92-94°C |
| 4 | $(CH_3)_3C$ | $CH_3$ | $-CH_2-CH=CH_2$ | 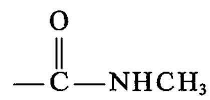 | S | zähes Öl |
| 5 | $CH_3$ | $CH_3$ | 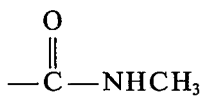 | $C_2H_5$ | S | $n_D^{20}$: 1,5232 |
| 6 | $CH_3$ | $C_2H_5$ | 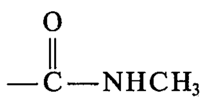 | $CH_3$ | S | $n_D^{20}$: 1,5165 |
| 7 | $CH_3$ | $C_2H_5$ | 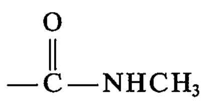 | $C_2H_5$ | S | $n_D^{20}$: 1,5114 |
| 8 | $CH_3$ | $CH_3$ | $CH_3$ | 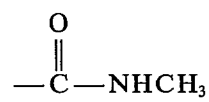 | S | $n_D^{20}$: 1,5280 |
| 9 | $CH_3$ | $CH_3$ | $C_2H_5$ | 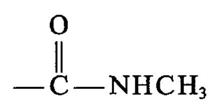 | S | $n_D^{20}$: 1,5207 |
| 10 | $-(CH_2)_3-$ | | 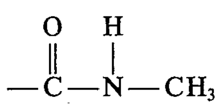 | $CH_3$ | S | $n_D^{20}$: 1,5478 |
| 11 | $(CH_3)_3C$ | $CH_3$ | 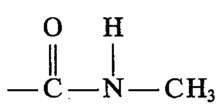 | $CH_3$ | S | zähes Öl |

Beispiel A

Phaedon-Larven-Test

Lösungsmittel: 3 Gewichtsteile Dimethylformamid
Emulgator: 1 Gewichsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das

Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen z. B. die folgenden Verbindungen der Herstellungsbeispiele überlegene Wirksamkeit gegenüber dem Stand der Technik: 1, 2, 3, 6, 9.

## Beispiel B

### Laphygma-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z. B. die folgenden Verbindungen der Herstellungsbeispiele überlegene Wirksamkeit gegenüber dem Stand der Technik: 1, 2, 3, 6.

## Beispiel C

### Tetranychus-Test (resistent)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 6.

## Beispiel D

### Grenzkonzentrations-Test/Bodeninsekten

Testinsekt: Phorbia antiqua (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 2, 3.

**Patentansprüche**

1. Hydroxamsäureester der Formel I

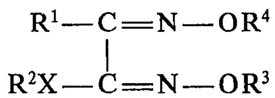

(I)

in welcher

$R^1$ für gegebenenfalls substituierte Alkyl, Cycloalkyl, Aryl, Aralkyl steht,
$R^2$ für Alkyl, Alkenyl, Alkinyl steht,
$R^1$ und $R^2$ gemeinsam für eine Alkylenbrücke mit 2–4 C-Atomen stehen und
einer der Reste $R^3$ oder $R^4$ für den Rest

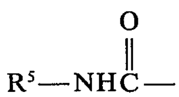

steht während der andere Rest $R^3$ oder $R^4$ für Alkyl, Alkenyl, Alkinyl, Halogenalkyl, Halogenalkenyl oder Alkoxyalkyl steht,
$R^5$ für Alkyl steht und
X für O oder S steht.

2. Verfahren zur Herstellung der Hydroxamsäureester der Formel I

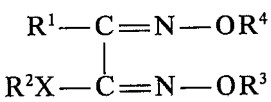

(I)

in welcher

$R^1$ für gegebenenfalls substituierte Alkyl, Cycloalkyl, Aryl, Aralkyl steht,
$R^2$ für Alkyl, Alkenyl, Alkinyl steht,
$R^1$ und $R^2$ gemeinsam für eine Alkylenbrücke mit 2–4 C-Atomen stehen und
einer der Reste $R^3$ und $R^4$ für den Rest

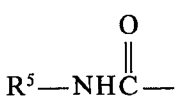

steht während der andere Rest $R^3$ oder $R^4$ für Alkyl, Alkenyl, Alkinyl, Halogenalkyl, Halogenalkenyl oder Alkoxyalkyl steht,
$R^5$ für Alkyl steht und
X für O oder S steht,

indem man Oxime der Formel II

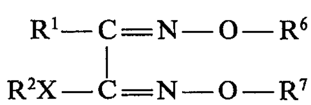

(II)

in welcher

$R^1$, $R^2$ und X die oben angegebenen Bedeutungen haben und
einer der Reste $R^6$ oder $R^7$ für H steht und der andere Rest $R^6$ oder $R^7$ für Alkyl, Alkenyl, Alkinyl, Halogenalkyl, Halogenalkenyl oder Alkoxyalkyl steht

mit Isocyanaten der Formel III

$R^5NCO$ (III)

in welcher

$R^5$ die oben angegebene Bedeutung hat,

umsetzt.

3. Oxime der Formel II

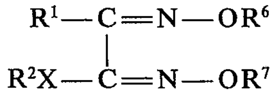

(II)

in welcher

$R^1$, $R^2$, X, $R^6$, $R^7$ die in Anspruch 2 angegebenen Bedeutungen haben.

4. Verfahren der Herstellung der Oxime der Formel II

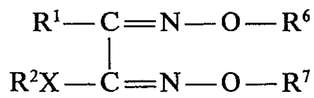

(II)

in welcher

$R^1$, $R^2$ und X die in Anspruch 1 angegebenen Bedeutungen haben und
einer der Reste $R^6$ oder $R^7$ für H steht und der andere Rest $R^6$ oder $R^7$ für Alkyl, Alkenyl, Alkinyl, Halogenalkyl, Halogenalkenyl oder Alkoxyalkyl steht,

indem man Ketone der Formel IV

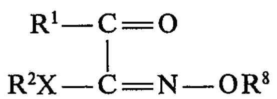

(IV)

in welcher

$R^1$, $R^2$ und X die in Anspruch 1 angegebenen Bedeutungen haben und
$R^8$ für Wasserstoff oder für die unter $R^7$ angegebenen Reste steht,

a) für den Fall, daß $R^8$ für Wasserstoff steht, mit Hydroxylaminethern bzw. deren Salzen der Formel V

$R^6ONH_2XHY$ (V)

in welcher

$R^6$ die oben angegebene Bedeutung hat und
Y für das Anion einer Säure steht,

umsetzt, oder

b) für den Fall, daß $R^8$ für die unter $R^7$ angegebenen Reste steht, mit Hydroxylamin bzw. seinen Salzen umsetzt.

5. Schädlingsbekämpfungsmittel, gekennzeichnet durch einen Gehalt an mindestens einem Hydroxamsäureester der Formel (I).

6. Verwendung von Hydroxamsäureester der Formel (I) zur Bekämpfung von Schädlingen.

7. Verfahren zur Bekämpfung von Schädlingen, dadurch gekennzeichnet, daß man Hydroxamsäureester der Formel (I) auf Schädlinge und/oder ihren Lebensraum einwirken läßt.

8. Verfahren zur Herstellung von Schädlingsbekämpfungsmitteln, dadurch gekennzeichnet, daß man Hydroxamsäureester der Formel (I) mit Streckmitteln und/oder oberflächenaktiven Mitteln vermischt.

## Claims

1. Hydroxamic acid esters of the formula I

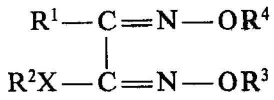

(I)

in which

$R^1$ represents optionally substituted alkyl, cycloalkyl, aryl or aralkyl and
$R^2$ represents alkyl, alkenyl or alkinyl, or
$R^1$ and $R^2$ together represent an alkylene bridge with 2–4 C atoms,
one of the radicals $R^3$ or $R^4$ represents the radical

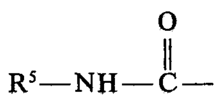

whilst the other radical $R^3$ or $R^4$ represents alkyl, alkenyl, alkinyl, halogenoalkyl, halogenoalkenyl or alkoxyalkyl,
$R^5$ represents alkyl and
X represents O or S.

2. Process for the preparation of the hydroxamic acid esters of the formula I

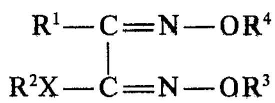

(I)

in which

$R^1$ represents optionally substituted alkyl, cycloalkyl, aryl or aralkyl and
$R^2$ represents alkyl, alkenyl or alkinyl, or
$R^1$ and $R^2$ together represents an alkylene bridge with 2–4 C atoms,
one of the radicals $R^3$ or $R^4$ represents the radical

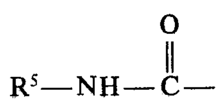

whilst the other radical $R^3$ or $R^4$ represents alkyl, alkenyl, alkinyl, halogenoalkyl, halogenoalkenyl or alkoxyalkyl,
$R^5$ represents alkyl and
X represents O or S,

by a procedure in which oximes of the formula II

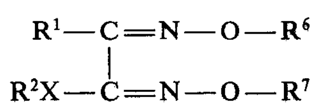

(II)

in which

$R^1$, $R^2$ and X have the abovementioned meanings and
one of the radicals $R^6$ or $R^7$ represents H and the other radical $R^6$ or $R^7$ represents alkyl, alkenyl, alkinyl, halogenoalkyl, halogenoalkenyl or alkoxyalkyl,

are reacted with isocyanates of the formula III

$$R^5NCO \quad (III)$$

in which

$R^5$ has the abovementioned meaning.

3. Oximes of the formula II

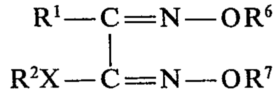

(II)

in which

$R^1$, $R^2$, X, $R^6$ and $R^7$ have the meanings given in Claim 2.

4. Process for the preparation of the oximes of the formula II

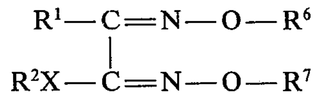

(II)

in which

$R^1$, $R^2$ and X have the meanings given in Claim 1 and
one of the radicals $R^6$ or $R^7$ represents H and the other radical $R^6$ or $R^7$ represents alkyl, alkenyl, alkinyl, halogenoalkyl, halogenoalkenyl or alkoxyalkyl,

by a procedure in which ketones of the formula IV

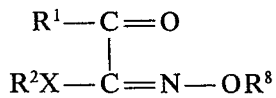

(IV)

in which

$R^1$, $R^2$ and X have the meanings given in Claim 1 and
$R^8$ represents hydrogen or the radicals given under $R^7$,

a) in the case where $R^8$ represents hydrogen, are reacted with hydroxylamine ethers, or salts thereof, of the formula V

$R^6ONH_2XHY$ (V)

in which

$R^6$ has the abovementioned meaning and
Y represents the anion of an acid, or

b) in the case where $R^8$ represents the radicals given unter $R^7$, are reacted with hydroxylamine or its salts.

5. Agents for combating pests, characterised in that they contain at least one hydroxamic acid ester of the formula (I).

6. Use of hydroxamic acid esters of the formula (I) for combating pests.

7. Process for combating pests, characterised in that hydroxamic acid esters of the formula (I) are allowed to act on pests and/or their environment.

8. Process for the preparation of agents for combating pests, characterised in that hydroxamic acid esters of the formula (I) are mixed with extenders and/or surface-active agents.

**Revendications**

1. Esters d'acides hydroxamiques de formule I

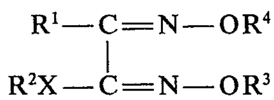

(I)

dans laquelle

$R^1$ représente un radical alkyle, cycloalkyle, aryle ou aralkyle éventuellement substitués,
$R^2$ représente un radical alkyle, alcényle, ou alcynyle,
$R^1$ et $R^2$ représentent, pris ensemble, un pont alkylène ayant 2 à 4 atomes de carbone, et
l'un des radicaux $R^3$ ou $R^4$ représente le radical

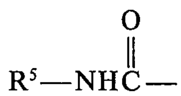

cependant que l'autre radical $R^3$ ou $R^4$ représente un radical alkyle, alcényle, alcynyle, halogénoalkyle, halogénoalcényle ou alcoxyalkyle,
$R^5$ représente un radical alkyle et
X représente O ou S.

2. Procédé de préparation des esters d'acides hydroxamiques de formule I

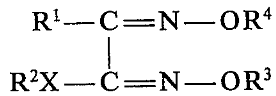

(I)

dans laquelle

$R^1$ représente un radical alkyle, cycloalkyle, aryle ou aralkyle éventuellement substitués,
$R^2$ représente un radical alkyle, alcényle ou alcynyle,
$R^1$ et $R^2$ représentent, lorsqu'ils sont pris ensemble, un pont alkylène ayant 2 à 4 atomes de carbone, et
l'un des radicaux $R^3$ ou $R^4$ représente le radical

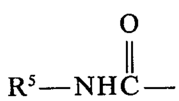

cependant que l'autre radical $R^3$ ou $R^4$ représente un radical alkyle, alcényle, alcynyle, halogénoalkyle, halogénoalcényle ou alcoxyalkyle,
$R^5$ représente un radical alkyle, et
X représente O ou S,

selon lequel on fait réagir des oximes de formule II

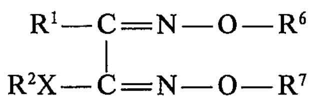

(II)

dans laquelle $R^1$, $R^2$ et X ont les sens indiqués ci-dessus, et l'un des radicaux $R^6$ et $R^7$ représente H et l'autre radical $R^6$ ou $R^7$ représente un radical alkyle, alcényle, alcynyle, halogénoalkyle, halogénoalcényle ou alcoxyalkyle,

avec des isocyanates de formule III

$R^5NCO$ (III)

dans laquelle

$R^5$ a le sens indiqué ci-dessus.

3. Oximes de formule II

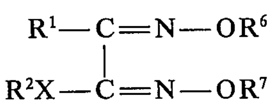

(II)

dans laquelle $R^1$, $R^2$, X, $R^6$, $R^7$ ont le sens indiqué à la revendication 2.

4. Procédé de préparation des oximes de formule II

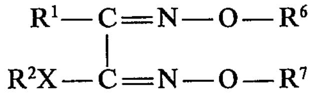

(II)

dans laquelle

$R^1$, $R^2$ et X ont les sens indiqués dans la revendication 1, et
l'un des radicaux $R^6$ ou $R^7$ représente H et l'autre radical $R^6$ ou $R^7$ représente un radical alkyle, alcényle, alcynyle, halogénoalkyle, halogénoalcényle ou alcoxyalkyle,

selon lequel on fait réagir des cétones de formule IV

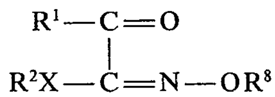

(IV)

dans laquelle

$R^1$, $R^2$ et X ont les sens indiqués à la revendication 1, et
$R^8$ représente un atome d'hydrogène ou les radicaux indiqués pour $R_7$,

a) si $R^8$ représente un atome d'hydrogène, avec des éthers d'hydroxylamines ou leurs sels de formule V

$R^6ONH_2XHY$ (V)

dans laquelle $R^6$ a le sens indiqué ci-dessus et Y représente l'anion d'un acide, ou

b) si $R^8$ représente les radicaux indiqués pour $R^7$, avec l'hydroxylamine ou ses sels.

5. Pesticide, caractérisé en ce qu'il contient au moins un ester d'acide hydroxamique de formule (I).

6. Application des esters d'acides hydroxamiques de formule (I) à la lutte contre les parasites.

7. Procédé de lutte contre les parasites, caractérisé en ce qu'on fait agir des esters d'acides hydroxamiques de formule (I) sur les parasites et/ou sur leur espace vital.

8. Procédé de préparation de pesticides, caractérisé en ce qu'on mélange des esters d'acides hydroxamiques de formule (I) avec des agents d'allongement et/ou des agents surfactifs.